# EUROPÄISCHE PATENTSCHRIFT

(11) **EP 2 023 829 B1**
(45) Veröffentlichungstag und Bekanntmachung des Hinweises auf die Patenterteilung: **09.09.2015**
(21) Anmeldenummer: 07764531.5
(22) Anmeldetag: 10.05.2007
(51) Int. Cl.: A61B 17/22, G10K 15/04, B01D 19/00, A61H 23/00

(54) **AUFBEREITUNGSVORRICHTUNG FÜR EINE VORRICHTUNG ZUR ERZEUGUNG VON STOSSWELLEN**
TREATMENT DEVICE FOR A DEVICE FOR PRODUCING SHOCK WAVES
DISPOSITIF DE TRAITEMENT POUR DISPOSITIF DE PRODUCTION D'ONDES DE CHOC

(30) Priorität: 13.05.2006 DE 102006022417
(43) Veröffentlichungstag der Anmeldung: 18.02.2009
(73) Patentinhaber: Storz Medical AG, 8274 Tägerwilen (CH)
(72) Erfinder: SCHWARZE, Werner, 07743 Jena (DE); STEPHAN, Hugo, 07745 Jena (DE)
(74) Vertreter: Lohr, Georg
(86) Internationale Anmeldenummer: PCT/EP2007/004156
(87) Internationale Veröffentlichungsnummer: WO 2007/131703

(56) Entgegenhaltungen:
- EP-A- 0 090 138
- DE-A1- 4 315 520
- DE-A1- 10 310 395
- FR-A- 2 663 239

## Beschreibung

Die Erfindung bezieht sich auf eine Vorrichtung zur Erzeugung und Fokussierung von Stoßwellen, insbesondere zur medizinischen Anwendung nach dem Oberbegriff des Patentanspruches 1.

Solche Vorrichtungen werden in der Medizintechnik, beispielsweise in der Urologie als Nierensteinzertrümmerer, in der Orthopädie bei nicht heilenden Knochenbrüchen oder bei Sehnenansatzendinosen oder ganz allgemein zur Förderung der Wundheilung seit Jahrzehnten eingesetzt. Um zum Beispiel im menschlichen Organ befindliche Nierensteine von außen durch akustische Stoßwellen zerstören zu können, ist es erforderlich, eine Vielzahl von Stoßwellen zu generieren und diese derart zu fokussieren, dass die Nierensteine in einem bestimmten Bereich der Stoßwellen angeordnet sind.

Die EP 0 090 138 A2 offenbart eine Vorrichtung zum Zerkleinern von im Körper eines Lebewesens befindlichen Konkrementen mit einer Fokussierungskammer, die Teil eines Rotationsellipsoids ist und in deren einem Brennpunkt Stoßwellen durch Funkenentladung erzeugbar sind, wobei der Körper in einer mit Wasser gefüllten Wanne liegt.

Die DE 43 15 520 A1 offenbart ein Verfahren und eine Vorrichtung zum katalytischen Entfernen von Sauerstoff aus Wasser, wobei Wasserstoff in das Wasser eingeleitet und dieses sodann durch ein Katalysatorbett hindurch geleitet wird.

Die FR 2 663 239 A1 offenbart im wesentlichen ein Verfahren zur Entgasung einer Kupplungsflüssigkeit einer Druckwellen-Generatorvorrichtung mittels Kupplungsflüssigkeiten auf chemischem Weg, eine so behandelte Kupplungsflüssigkeit und einen Druckwellen-Generator, bei dem eine derartige Kupplungsflüssigkeit verwendet wird.

Die Stosswellen werden von verschiedenen elektrohydraulischen, elektromagnetischen oder piezoelektrischen Systemen, vorzugsweise in Wasser, erzeugt. Der Grund hierfür ist, dass die Stosswellen nach ihrer Erzeugung in den zu therapierenden Körper eingekoppelt werden müssen und dass das zu durchdringende Weichgewebe des Körpers vergleichbare akustische Eigenschaften wie Wasser besitzt. Gleichzeitig dient das Wasser zur Ableitung der entstehenden Prozesswärme bzw. zur Spülung des Elektodenabbrandes während der Erzeugung der Stosswelle. Ferner kann über eine Koppelmembran und unterschiedliche Wasserdrücke in der Membran die Ankopplung an unterschiedliche Körperkonturen und -dicken erreicht werden.

Zur Erzeugung der Stoßwelle wird z.B. in der Vorrichtung ein Elektrodenpaar verwendet, zwischen denen durch Funkenentladung die Stoßwellen erzeugt werden. Darüber hinaus ist es erforderlich, die Elektroden in Wasser unterzubringen, denn das Wasser dient für die Stoßwellen als Weiterleitungs- und Kopplungsmedium. Ähnliches gilt für elektromagnetische oder piezoelektrische Systeme. Üblicherweise ist die Fokussiereinrichtung, durch die die Stoßwellen ausgerichtet werden, als sphärischer Reflektor oder als Linsensystem ausgebildet. Auch in der Fokussiereinrichtung ist als Weiterleitungs- und Kopplungsmedium Wasser vorzusehen, um die Stoßwellen aus der Fokussiereinrichtung weiterzuleiten. Die Fokussiereinrichtung ist in Richtung des Behandlungszentrums durch eine Membrane oder einen Balg flüssigkeitsdicht verschlossen, so dass das Wasser innerhalb der Fokussiereinrichtung verbleibt.

In dem verwendeten Wasser sind jedoch auf natürliche Weise Gase, die in der Atmosphäre vorkommen, nämlich O₂, N₂ und CO₂ gelöst. Das Verhältnis der gelösten Gase O₂ zu N₂ ist in Wasser bei Zimmertemperatur etwa 4 zu 1, wobei der Anteil des gelösten CO₂ vernachlässigbar ist, so dass der gelöste Sauerstoff prozentual am häufigsten vorkommt. Ist der Wasserkreislauf offen bzw. nicht diffusionsdicht für diese Gase, wird sich das Wasser des Kreislaufes im Laufe der Zeit auch immer wieder begasen. Zusätzlich entstehen bei der elektrohydraulischen Stosswellenerzeugung während des Funkensprungs durch Stossionisation Sauerstoff und Wasserstoff. Selbst wenn der Wasserstoff durch Diffusion aus dem System entweicht, bleibt der Sauerstoff gelöst oder gasförmig im System zurück.

Die gelösten Gase beeinträchtigen jedoch die Stosswellenausbreitung, da sie im Zugwellenanteil der Stosswellen zu Kavitationsblasen führen. Deren Entstehung verbraucht Energie und die im Schallfeld befindlichen mikroskopischen Gasblasen behindern durch Streuung und Reflektion die Stosswellenausbreitung.

Als Konsequenz ist eine Reduktion des Gerätewirkungsgrades festzustellen. Zur Vermeidung dieses Verlustes werden spezielle Entgasungsvorrichtungen in den Wasserkreislauf eingebaut. Diese funktionieren zumeist durch Erhitzen, Anlegen einer Unterdruckatmosphäre, Versprühen des Wassers in die Unterdruckatmosphäre oder auch durch Einsatz semipermeabler Membranen. Anschließend wird das derart entgaste Wasser zurück in die Fokussiereinrichtung geführt. All diese Systeme sind aufwendig, teuer, großvolumig und benötigen spezielle Pumpensysteme müssen gasdicht ausgebildet sein und erreichen im Ergebnis nur Werte von größer 1,0 bis 1,5 Mikrogramm gelöstem O₂ pro Liter Wasser. Erstrebenswert sind Werte deutlich kleiner , beispielsweise 1,0 Mikrogramm gelöstem O₂ pro Liter Wasser

Aus diesen Gründen ist es üblich, den Wasserkreislauf mittels Unterdruck zu entgasen. Das Wasser strömt demnach aus der Leitung in einen Behälter, in dem die Sauerstoffmoleküle dem Wasser durch Unterdruck entzogen werden. Anschließend wird das derart entgaste Wasser zurück in die Fokussiereinrichtung durch die Leitung geführt.

Als nachteilig hat sich bei der Entgasung mittels Unterdruck herausgestellt, dass die Behälter gasdicht ausgebildet sein müssen, um einen genügend hohen Unterdruck im Inneren des Behälters erzeugen zu können, damit die Sauerstoffmoleküle aus dem Wasser entweichen können. Eine solche Behälterausbildung ist jedoch äußerst kostenintensiv herzustellen. Darüber hinaus muß das gesamte System gasdicht ausgebildet sein.

Es ist daher Aufgabe der Erfindung eine Aufbereitungsvorrichtung der eingangs genannten Gattung derart weiterzubilden, dass die in dem Wasser natürlich vorkommenden Gase bzw. das durch die Stoßwellenerzeugung entstehende Gas chemisch gebunden bzw. in Wasser zurückverwandelt wird. Hierzu ist es z.B. notwendig, dass möglichst wenig Wasserstoffmoleküle aus dem Wasserkreislauf entweichen und somit weiterhin als Reaktionspartner für die Sauerstoffmoleküle zur Bildung von Wasser vorhanden sind.

Diese Aufgabe wird erfindungsgemäß durch die Merkmale des kennzeichnenden Teiles des Patentanspruches 1 gelöst.

Weitere vorteilhafte Weiterbildungen der Erfindung ergeben sich aus den Unteransprüchen.

Um den natürlich vorkommenden, gelösten Sauerstoff chemisch zu entfernen, bieten sich vor allem Reduktionsreaktionen an. Z.B. kann Natriumsulfit durch Aufnahme von Sauerstoff in Natriumsulfat umgewandelt werden. Beide Salze sind wasserlöslich und nicht toxisch, eignen sich also dementsprechend für den Einsatz in medizinischen Systemen.

Eine weitere erfindungsgemäße Ausführung verhindert durch ein Speichermedium im Wasser, dass die bei der Stoßwellenerzeugung entstehenden Wassermoleküle entweichen. Das Speichermedium kann im Wasser gelöst, suspendiert oder in Form eines durchströmte Reaktionsbehälters im Wasserkreislauf vorhanden sein.

Dadurch, dass das Speichermedium die in dem Wasser vorhandenen frei beweglichen Wasserstoffmoleküle bindet und diese somit in der Flüssigkeit gehalten werden, stehen die Wasserstoffmoleküle für die frei beweglichen Sauerstoffmoleküle weiterhin als Reaktionspartner zur Verfügung. Durch die derart eingeleitete chemische Reaktionsfolge entsteht aus dem Wasserstoff- und den Sauerstoffmolekülen Wasser.

Zudem ist der Behälter nicht gasdicht auszubilden und zu verschließen, denn in diesem findet lediglich eine chemische Reaktion zwischen den Wasserstoffmolekülen und den Sauerstoffmolekülen statt, wodurch die Herstellungskosten für den Behälter reduziert sind.

Um den freien Sauerstoffmolekülen genügend Reaktionspartner in Form von Wasserstoffmolekülen zur Verfügung zu stellen, können z.B. Hydrazin oder wässrige organische oder anorganische Säuren dem Wasserkreislauf zugegeben werden. Diese Stoffe geben permanent Wasserstoffmoleküle an die in den Behälter einströmende Flüssigkeit ab, so dass für die in der Flüssigkeit vorhandenen Sauerstoffmoleküle genügend Reaktionspartner vorhanden sind.

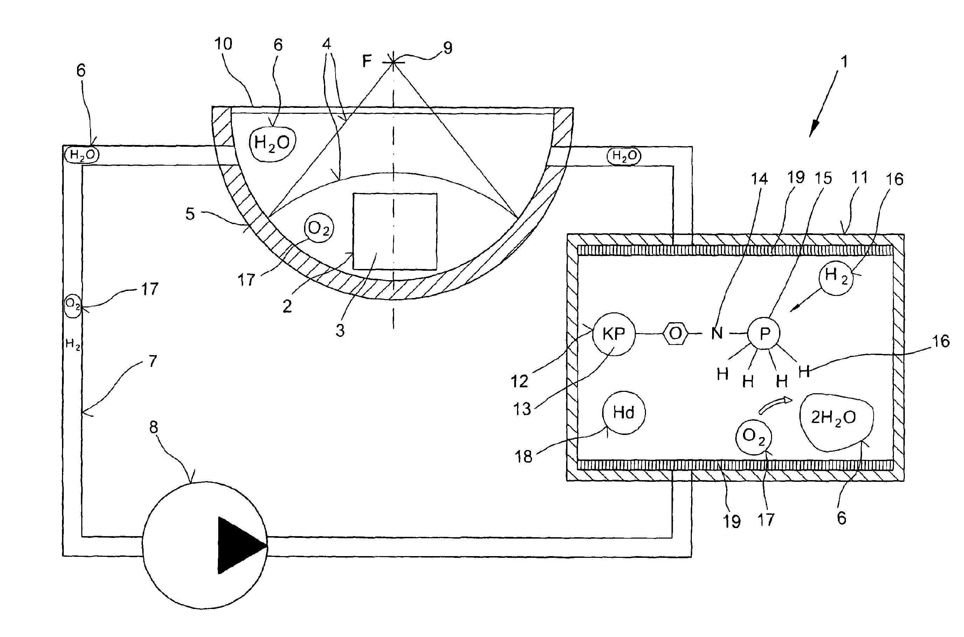

In der einzigen Zeichnung ist ein erfindungsgemäßes Ausführungsbeispiel dargestellt, das nachfolgend erläutert wird. Die Figur zeigt eine Aufbereitungsvorrichtung für eine Vorrichtung zur Erzeugung von Stoßwellen und für eine Fokussiereinrichtung, in die die Erzeugungsvorrichtung eingesetzt ist und in der als Übertragungsmedium für die Stoßwellen Wasser eingefüllt ist sowie mit einer als Wasserkreislauf ausgebildete Leitung zur Aufbereitung des Wassers in einem Behälter.

Eine Aufbereitungsvorrichtung 1, die zur chemischen Entgasung von Wasser 6 verwendet wird, soll einer Vorrichtung 2 zur Erzeugung von Stoßwellen 4 zugeordnet sein. Die Vorrichtung 2 zur Erzeugung der Stoßwellen 4 besteht aus einem Gehäuse 3, in dem beispielsweise durch nicht dargestellte Elektrodenpaare die Stoßwellen 4 über einen Spannungsdurchbruch im Wasser generiert werden. Die Stoßwellen 4 werden mittels eines Übertragungs- und Kopplungsmediums in Form von Wasser 6 nach außen abgegeben. Folglich ist sowohl das Gehäuse 3 als auch das das Gehäuse 3 umgebende Volumen mit Wasser 6 vollständig gefüllt.

Damit das in der Fokussiereinrichtung 5 eingefüllte Wasser 6 nicht aus dieser ausfließen kann, ist diese mittels einer Membrane 10 in Richtung des Behandlungszentrums 9 wasserdicht verschlossen. Die Außenseite der Membrane 10 wird an den zu behandelnden Körper angekoppelt. Dieser besteht bekanntermaßen zum überwiegenden Anteil aus Wasser, so dass die Stoßwellen 4 im menschlichen Körper nahezu ungehindert weitergeleitet werden.

Neben der natürlichen initialen Begasung des Wassers durch gelöste Gase aus der Atmosphäre hat sich des Weiteren gezeigt, dass sich bei der elektrohydraulischen Stosswellenerzeugung aufgrund der in dem Gehäuse stattfindenden elektrochemischen Reaktion in Form von Stoßionisation freie Wasserstoff- und Sauerstoffmoleküle 16 und 17 bilden. Die Wasserstoffmoleküle 16 diffundieren nach kürzester Zeit ungehindert in die Atmosphäre und die Sauerstoffmoleküle 17 bilden Gasblasen und lagern sich im Wasser 6 selbst an der Membrane 10, der Fokussiereinrichtung 5 oder dem Gehäuse 3 ab und behindern somit die Ausbreitung der Stoßwellen 4.

Um diese Ablagerung von Gasen, insbesondere von Sauerstoffmolekülen 17, zu verhindern, wird das Wasser 6 und damit auch die Sauerstoffmoleküle 17 aus der Fokussiereinrichtung 5 durch eine Leitung 7 abgeführt. In der Leitung 7 ist eine Pumpe 8 vorgesehen, durch die der Volumenstrom zur Umwälzung des Wassers 6 einstellbar ist.

Die Leitung 7 mündet in einen Behälter 11, in den das Wasser 6 somit einströmt. Im 15 Inneren des Behälters 11 soll eine chemische Entgasung vollzogen werden, und die Sauerstoffmoleküle 17 mit Wasserstoff in Wasser 6 umzuwandeln. Daher sind in den Behälter 11 eine Vielzahl eines Speichermediums 12 eingefüllt, die schematisch dargestellt werden. Das Speichermedium 12 besteht z.B. aus einer Kunstharzperle 13, an der über eine Stickstoffverbindung 14 ein Paladiumpartikel 15, das eine metallisierte Oberfläche aufweist, angebracht ist. Die frei beweglichen Wasserstoffmoleküle 16 bzw. wasserstoffabgebende Stoffe wie Hydrazin oder Säuren strömen an den Paladiumpartikel 15 vorbei und werden durch diesen gebunden.

Es erfolgen drei chemische Reaktionsschritte zwischen der metallisierten Außenschicht der Paladium-Partikel 15 und dem im Wasser 6 vorhandenen Wasserstoffmolekülen 16;

1. H₂ + PdO → PdO + 2H.

Sobald die im Wasser 6 vorhandenen Sauerstoffmoleküle 17 entlang der derart an dem Paladiumpartikel 15 angebrachten Wasserstoffmolekülen 16 vorbeiströmen, findet eine weitere chemische Reaktion statt, nämlich

2H + O-O → HO-OH,

diese Verbindung reagiert weiter nach der chemischen Formel

2H˙ + HO-OH → 2H₂O

Durch diesen Reaktionsablauf hat sich folglich aus den durch die Stoßionisation freigesetzten Wasserstoff- und Sauerstoffmolekülen 16 und 17 erneut Wasser 6 gebildet.

Sobald das in den Behälter 11 vorhandene Wasser 6 chemisch entgast ist, wird dieses über die Leitung 7 zurück in die Fokussiereinrichtung 5 geleitet.

2H˙ + ˙O-O˙ → 2HO-OH,

diese Verbindung reagiert weiter nach der chemischen Formel

2H˙ + 2HO-OH → 2H₂O

Durch diesen Reaktionsablauf hat sich folglich aus den durch die Stossionisation freigesetzten Wasserstoff- und Sauerstoffmolekülen 16 und 17 erneut Wasser 6 gebildet.

Sobald das in den Behälter 11 vorhandene Wasser 6 chemisch entgast ist, wird dieses über die Leitung 7 zurück in die Fokussiereinrichtung 5 geleitet.

## Patentansprüche

1. Aufbereitungsvorrichtung (1)
mit einer Vorrichtung (2) zur Erzeugung von Stosswellen (4), die insbesondere zur medizinischen Anwendung einsetzbar sind,
mit einem Gehäuse (3), in dem die Vorrichtung (2) zur Erzeugung der Stosswellen (4) angeordnet ist,
mit einer Fokussiereinrichtung (5), in die das Gehäuse (3) eingesetzt ist und die durch eine Membran (10) oder einen Balg flüssigkeitsdichtend nach außen verschlossen ist,
mit einer in der Fokussiereinrichtung (5) eingefüllten Flüssigkeit (6), die die Vorrichtung (2) zur Erzeugung der Stosswellen (4) und das Gehäuse (3) umschließt, und
mit einer Leitung (7), durch die die Flüssigkeit (6) der Fokussiereinrichtung (5) zuführbar und aus dieser ableitbar ist,
**dadurch gekennzeichnet, dass**
die Leitung (7) an einen Behälter (11) angeschlossen ist, in dem die Flüssigkeit (6) derart chemisch entgasbar ist, dass die in der Flüssigkeit (6) vorhandenen Sauerstoffmoleküle (17) mit Wasserstoffmolekülen (16) zu Wasser (6) oder mit einem Reduktionsmittel reagieren, und
dass der Behälter (11) nicht gasdicht ausgebildet und verschlossen ist.

2. Vorrichtung nach Anspruch 1,
**dadurch gekennzeichnet, dass**
in dem Behälter (11) eine Vielzahl von Speichermedien (12) eingefüllt sind, durch die Wasserstoffmoleküle (16) gebunden sind.

3. Vorrichtung nach Anspruch 2,
**dadurch gekennzeichnet, dass**
die Speichermedien (12) jeweils aus Kunstharzperlen (13) bestehen, deren Oberfläche Nano- oder Mikropartikel-Strukturen aufweist, an denen über eine oder mehrere Stickstoffverbindungen (14) eine oder mehrere Palladiumpartikel (15) angebracht sind.

4. Vorrichtung nach Anspruch 3,
**dadurch gekennzeichnet, dass**
die Palladiumpartikel (15) eine metallisierte Oberfläche aufweisen, an denen Wasserstoffmoleküle (16) chemisch arretierbar sind .

5. Vorrichtung nach einem oder mehreren der vorgenannten Ansprüche,
**dadurch gekennzeichnet, dass**
in dem Behälter (11) ein chemischer Stoff (18) eingefüllt, nachfüllbar oder zudosierbar ist, von dem Wasserstoffmoleküle (16) auf die durch den Behälter (11) strömende Flüssigkeit (6) übertragbar sind.

6. Vorrichtung nach Anspruch 5,
**dadurch gekennzeichnet, dass**
als chemischer Stoff (18) Hydrazin oder verdünnte Säuren einsetzbar sind.

7. Vorrichtung nach einem oder mehreren der vorgenannten Ansprüche,
**dadurch gekennzeichnet, dass**
die Innenseiten des Behälters (11) im Bereich der Eintritts- und der Austrittsöffnung der Leitungen (7) mit einem Vlies (19), einer Membran oder einem Sieb abgedeckt ist, die von dem Speichermedium (12) und den Wasserstoffmolekülen (17) undurchdringbar sind.

8. Vorrichtung nach einem oder mehreren der vorgenannten Ansprüche,
**dadurch gekennzeichnet, dass**
die Fokussiereinrichtung (5) und/oder die Leitung (7) mit einer aus den Speichermedien (12) gebildeten Barriereschicht versehen ist bzw. sind, die für Wasserstoffmoleküle (16) undurchlässig ist.

9. Vorrichtung nach einem oder mehreren der vorgenannten Ansprüche,
**dadurch gekennzeichnet, dass**
in der Leitung (7) eine Pumpe (8) angeordnet ist, mittels der die Flüssigkeit (6) durch den Behälter (11) förderbar ist.

10. Vorrichtung nach Anspruch 1,
**dadurch gekennzeichnet, dass**
sowohl das Reduktionsmittel als auch das Reaktionsprodukt wasserlöslich ist.

11. Vorrichtung nach Anspruch 1,
**dadurch gekennzeichnet, dass**
das Reduktionsmittel im gesamten Wasserkreislauf (5, 11) vorhanden ist.

12. Vorrichtung nach Anspruch 1,
**dadurch gekennzeichnet, dass**
das Reduktionsmittel, ein nichttoxischer Stoff, vorzugsweise Natriumsulfit, ist.

## Claims

1. Treatment device (1)
with a device (2) for generating shock waves (4), which are applicable in particular for medical use,
with a housing (3), in which the device (2) for generating the shock waves (4) is arranged,
with a focusing device (5), in which the housing (3) is inserted and which is closed fluid-tightly by a membrane (10) or a bellows towards the outside,
with a fluid (6) filled in the focusing device (5), which encloses the device (2) for generating the shock waves (4) and the housing (3), and
with a conduit (7), through which the fluid (6) can be fed into and drained off the focusing device (5),
**characterized in that**
the conduit (7) is connected to a container (11), in which the fluid (6) is chemically degassable, such that the oxygen molecules (17) present in the fluid (6) react with hydrogen molecules (16) to water (6), or react with a reducing agent, and
that the container (11) is formed and closed non-gas-tightly.

2. Device according to claim 1
**characterized in that**
a plurality of storage media (12) is filled into the container (11), by which storage media (12) hydrogen molecules (16) are bound.

3. Device according to claim 2,
**characterized in that**
the storage media (12) are each made of synthetic resin beads (13), the surface of said synthetic resin beads (13) comprising nano- or microparticle-structures, at which one or more palladium particles (15) are attached via one or more nitrogen bonds (14).

4. Device according to claim 3,
**characterized in that**
the palladium particles (15) comprise a metallized surface, at which surface hydrogen molecules (16) can be chemically locked.

5. Device according to one or more of the preceding claims,
**characterized in that**
a chemical substance (18) is filled, refillable, or can be dosed in the container (11), from which chemical substance hydrogen molecules (16) are transferable to the fluid (6) flowing through the container (11).

6. Device according to claim 5,
**characterized in that**
hydrazine or diluted acids are used as chemical substance (18).

7. Device according to one or more of the preceding claims,
**characterized in that**
the inner sides of the container (11) are covered with a fleece (19), a membrane or a sieve in the region of the inlet and the outlet opening of the conduits (7), said fleece (19), membrane or sieve are impermeable for the storage medium (12) and the hydrogen molecules (17).

8. Device according to one or more of the preceding claims,
**characterized in that**
the focusing device (5) and/or the conduit (7) is/are provided with a barrier layer formed of the storage media (12), which barrier layer is impermeable for hydrogen molecules (16).

9. Device according to one or more of the preceding claims,
**characterized in that**
a pump (8) is arranged in the conduit (7), by means of which the fluid (6) can be conveyed through the container (11).

10. Device according to claim 1,
**characterized in that**
the reducing agent as well as the reaction product is water soluble.

11. Device according to claim 1,
**characterized in that**
the reducing agent is present in the entire water circuit (5, 11).

12. Device according to claim 1,
**characterized in that**
the reducing agent is a non-toxic substance, preferably sodium sulfite.

## Revendications

1. Dispositif de traitement (1)
avec un dispositif (2) pour la génération d'ondes de choc (4) qui peuvent être utilisées en particulier pour un usage médical,
avec un boîtier (3) dans lequel est disposé le dispositif (2) pour la génération d'ondes de choc (4),
avec un dispositif de focalisation (5) dans lequel le boîtier (3) est inséré et qui est fermé par une membrane (10) ou un soufflet de manière étanche au liquide vis-à-vis de l'extérieur,
avec un liquide (6) remplissant le dispositif de focalisation (5), qui entoure le dispositif (2) pour la génération d'ondes de choc (4) et le boîtier (3), et
avec une conduite (7) par laquelle le liquide (6) peut être amené au dispositif de focalisation (5) et extrait de celui-ci,
**caractérisé en ce que**
la conduite (7) est raccordée à un réservoir (11) dans lequel le liquide (6) peut être dégazé chimiquement de telle façon que les molécules d'oxygène (17) présentes dans le liquide (6) réagissent avec des molécules d'hydrogène (16) en formant de l'eau (6) ou avec un agent réducteur, et **en ce que** le réservoir (11) n'est pas construit ou fermé de façon étanche au gaz.

2. Dispositif selon la revendication 1,
**caractérisé en ce que**
le réservoir (11) est rempli d'un grand nombre de supports d'accumulation (12) auxquels les molécules d'hydrogène (16) sont liées.

3. Dispositif selon la revendication 2,
**caractérisé en ce que**
les supports d'accumulation (12) se composent de perles de résine de synthèse (13) dont la surface présente des structures en nano- ou microparticules sur lesquelles une ou plusieurs particules de palladium (15) sont appliquées à l'aide d'un ou plusieurs liaisons d'azote (14).

4. Dispositif selon la revendication 3,
**caractérisé en ce que**
les particules de palladium (15) présentent une surface métallisée sur laquelle des molécules d'hydrogène (16) peuvent être retenues chimiquement.

5. Dispositif selon une ou plusieurs des revendications précédentes,
**caractérisé en ce que**
le réservoir (11) peut être rempli, complété ou additionné d'une substance chimique (18) à partir de laquelle des molécules d'hydrogène (16) peuvent être transférées au liquide (6) circulant à travers le réservoir (11).

6. Dispositif selon la revendication 5,
**caractérisé en ce que**
la substance chimique (18) utilisée peut être de l'hydrazine ou des acides dilués.

7. Dispositif selon une ou plusieurs des revendications précédentes,
**caractérisé en ce que**
les faces intérieures du réservoir (11) sont recouvertes, au niveau des ouvertures d'entrée et de sortie des conduites (7), avec un non-tissé (19), une membrane ou un crible qui est impénétrable au support d'accumulation (12) et aux molécules d'hydrogène (17).

8. Dispositif selon une ou plusieurs des revendications précédentes,
**caractérisé en ce que**
le dispositif de focalisation (5) et/ou la conduite (7) sont munis d'une couche barrière formée avec les supports d'accumulation (12), qui est impénétrable aux molécules d'hydrogène (16).

9. Dispositif selon une ou plusieurs des revendications précédentes,
**caractérisé en ce qu'**
est disposée dans la conduite (7) une pompe (8) au moyen de laquelle le liquide (6) peut être transporté à travers le réservoir (11).

10. Dispositif selon la revendication 1,
**caractérisé en ce que**
l'agent réducteur aussi bien que le produit de réaction sont hydrosolubles.

11. Dispositif selon la revendication 1,
**caractérisé en ce que**
l'agent réducteur est présent dans tout le circuit d'eau (5, 11).

12. Dispositif selon la revendication 1,
**caractérisé en ce que**
l'agent réducteur est une substance non toxique, de préférence du sulfite de sodium.
